# EUROPEAN PATENT APPLICATION

(11) **EP 3 912 985 A1**
(43) Date of publication of application: **24.11.2021**
(21) Application number: 20741441.8
(22) Date of filing: 19.01.2020
(51) Int. Cl.: C07J 43/00

(54) **CRYSTAL FORMS OF ANTIDEPRESSANT DRUG SAGE-217 AND PREPARATION METHOD THEREFOR**

(30) Priority: 20.01.2019 CN 201910050777
(71) Applicant: Suzhou Pengxu Pharmatech Co., Ltd., Suzhou 215200 (CN); Zhejiang Eazy Pharmchem Co., Ltd, Hangzhou, Zheijiang 310020 (CN)
(72) Inventor: WANG, Peng, Suzhou, Jiangsu 215200 (CN); LI, Pixu, Suzhou, Jiangsu 215200 (CN); CHEN, Siping, Hangzhou, Zhejiang 310020 (CN)
(74) Representative: Pawlyn, Anthony Neil
(86) International application number: PCT/CN2020/072924
(87) International publication number: WO 2020/147852

(57) **Abstract**

The present application relates to crystalline form 04, crystalline form 06, crystalline form D-1, and crystalline form D-2 of an antidepressant drug SAGE-217 and a preparation method therefor and a pharmaceutical composition containing same. The crystalline form 04 has an XRPD pattern with characteristic peaks at 2theta values of 11.6±0.2°, 13.5±0.2°, 16.2±0.2°, 16.5±0.2°, and 23.2±0.2°; the crystalline form 06 has an XRPD pattern with characteristic peaks at 2theta values of 8.7±0.2°, 10.0±0.2°, 13.2±0.2°, 15.0±0.2°, 15.8±0.2°, and 17.3±0.2°; the crystalline form D-1 has an XRPD pattern with characteristic peaks at 2theta values of 7.2±0.2°, 8.6±0.2°, 13.3±0.2°, 19.6±0.2°, and 23.0±0.2°; and the crystalline form D-2 has an XRPD pattern with characteristic peaks at 2theta values of 7.3±0.2°, 8.6±0.2°, 13.4±0.2°, 19.7±0.2°, and 23.3±0.2°. The novel crystalline forms provided by the present application have good stability, and provide more choices for drug development.

## Description

### Technical Field of the Invention

The present disclosure relates to the chemical pharmaceutical field, and in particular to crystalline forms of an antidepressant drug SAGE-217 and a preparation method therefor.

### Background of the Invention

SAGE-217 is a drug candidate for the treatment of symptoms such as postpartum depression and major depression. Existing antidepressant drugs as a single treatment often have limitations. SAGE-217 is a new generation of GABA receptor regulating drug, optimized for the selectivity of synaptic and extra-synaptic GABA receptors and the pharmacokinetic characteristics of daily oral administration. The GABA system is the main inhibitory signal pathway of the brain and central nervous system, and is of great significance for regulating the function of the central nervous system. The regulation of GABA receptors has a relatively obvious therapeutic effect.

The structure of SAGE-217 compound is shown in formula I, and the chemical name is 1-(2-((3R,5R,8R,9R,10S,13S,14S,17S)-3-hydroxy-3,13-dimethylhexadecahydro-1H-cyclopenta[a]phenanthren-17-yl)-2-oxoethyl)-1H-pyrazole-4- carbonitrile.

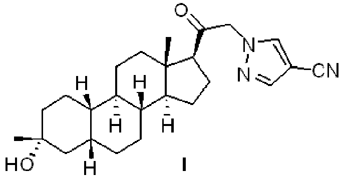

International patent application WO2018039378A1 describes the crystalline forms A, B, C, D, E, F, H, I, J, K, L, M, N, O and P of SAGE-217, and methods for preparing polymorphs.

The polymorphs A, C, and K described in the patent application are anhydrous crystalline forms, crystalline forms B, F, N, O, and P are solvates, crystalline forms L, M, H, I, and J are metastable state, and crystalline forms D and E are not clearly described.

The polymorphic form of a compound refers to the state of matter in which there are two or more different crystalline forms in the compound. Polymorphism exists extensively in organic compounds. The solvate is an important state of matter in polymorphism. Different crystalline forms of the same compound have significant differences in solubility, melting point, density, stability, etc., which affect the stability and uniformity of the compound to varying degrees. Different crystalline forms have obvious differences in the purification ability of the compound through crystallization in the purification process of the compound. Therefore, comprehensive and systematic polymorphic screening and selection of the most suitable crystalline form in the research and development of pharmaceutical processes are one of the important research contents that cannot be ignored.

### Summary of the Invention

This application is aimed to provide novel crystalline forms of SAGE-217 and a preparation method therefor.

In a specific implementation, this application relates to crystalline form 04, crystalline form 06, crystalline form D-1 and crystalline form D-2 of SAGE-217, which are mainly characterized by X-ray powder diffraction ("XRPD") data, and at the same time, it may also refer to the related data characterization of differential scanning calorimetry ("DSC"), thermogravimetric analysis ("TGA") and evolved gas analysis ("EGA").

The present disclosure further provides a method for preparing crystalline SAGE-217 through crystallization method. The SAGE-217 raw material used therein may be prepared by any suitable method, including synthetic methods known in the art.

A solution of the present application is: a crystalline form 04 of SAGE-217 has an X-ray powder diffraction pattern with characteristic peaks at 2theta values of 11.6±0.2°, 13.5±0.2°, 16.2±0.2°, 16.5±0.2°, and 23.2±0.2°.

Further, it has an X-ray powder diffraction pattern with characteristic peaks at 2theta values of 6.8±0.2°, 14.7±0.2°, 18.7±0.2°, 19.2±0.2°, and 21.3±0.2°.

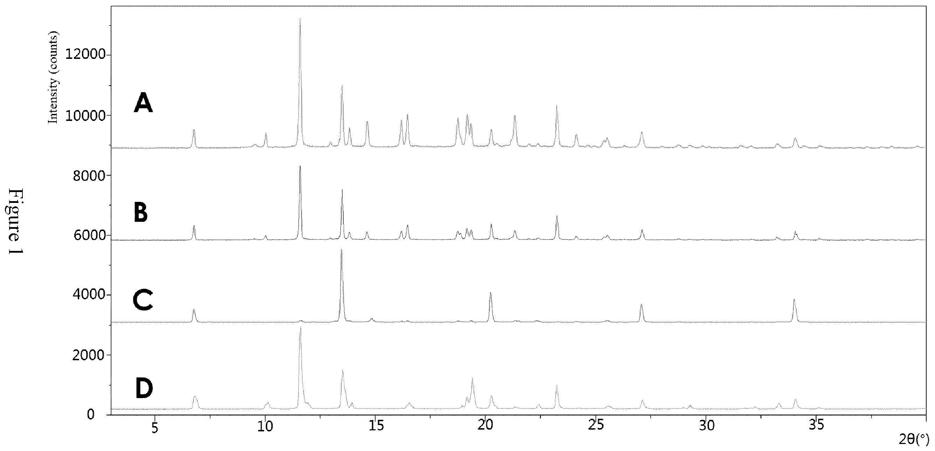

According to some specific implementations of this application, the crystalline form 04 has an X-ray powder diffraction pattern (peak positions) substantially as depicted in Figure 1.

The present disclosure further provides highly crystalline SAGE-217 crystalline form 04 that may be affected by the preferred orientation phenomenon, as shown in the exemplary XRPD patterns of C and D in Figure 1. Since the preferred orientation phenomenon is known to significantly change the relative intensity of some peaks in the XRPD pattern, for the crystalline form 04 crystalline powder with a high degree of orientation, if it is not properly processed before the measurement, it may be observed that the peak intensities of the 2theta value of 19.4±0.2°, 20.3±0.2°, 27.2±0.2° and 34.1±0.2° in the XRPD pattern are increased significantly.

In another aspect, this application provides a method for preparing the crystalline form 04 of SAGE-217 from a solution of ethyl formate or formic acid mixed with alcohol, dichloromethane or acetonitrile by cooling or evaporative crystallization.

In a specific implementation, the crystalline form 04 of crystalline SAGE-217 may be prepared by the methods reported in Examples 1 to 5.

The crystalline form 04 may be prepared by cooling or evaporative crystallization after it is completely dissolved in the formate or the mixed solvent of formic acid and alcohols. Generally, the chemical and physical stability of solvates is relatively poor. We unexpectedly found that after 9 months of storage at a temperature of 0 - 8 °C, the chemical stability of crystalline form 04 is good, the purity is basically unchanged, and the crystalline form remains. The crystalline form 04 remains stable in the exposed powder state for one day, and has a crystalline form stability of more than seven days when stored in a sealed vial at 25 °C/45% RH. Under the accelerated stability test conditions of 25 °C/60% humidity and 40 °C/75% humidity, the crystalline form may be stored stably for one month without change. The DVS curve of crystalline form 04 shows that crystalline form 04 has no hygroscopicity. More importantly, compared with the previously reported crystalline form K, the crystalline form 04 has better mechanical stress stability, that is, better mechanical processing performance. This will be of great significance to the application of crystalline form 04 in formulations.

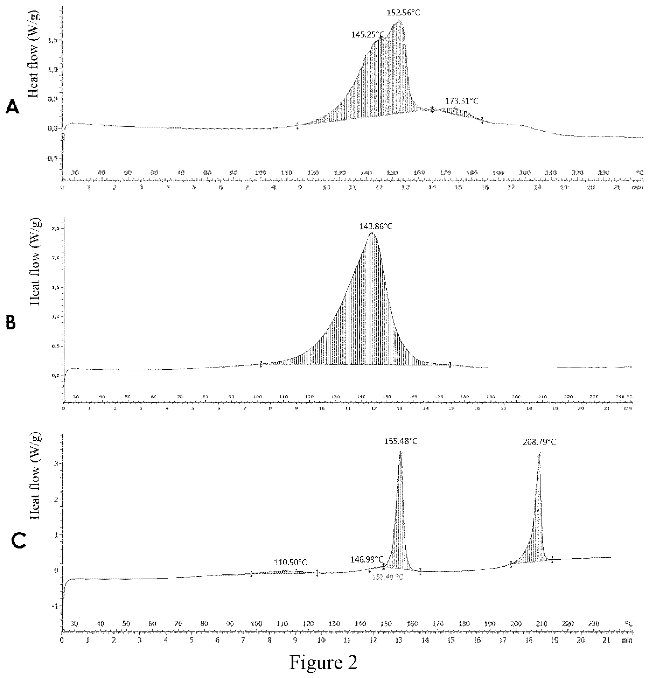

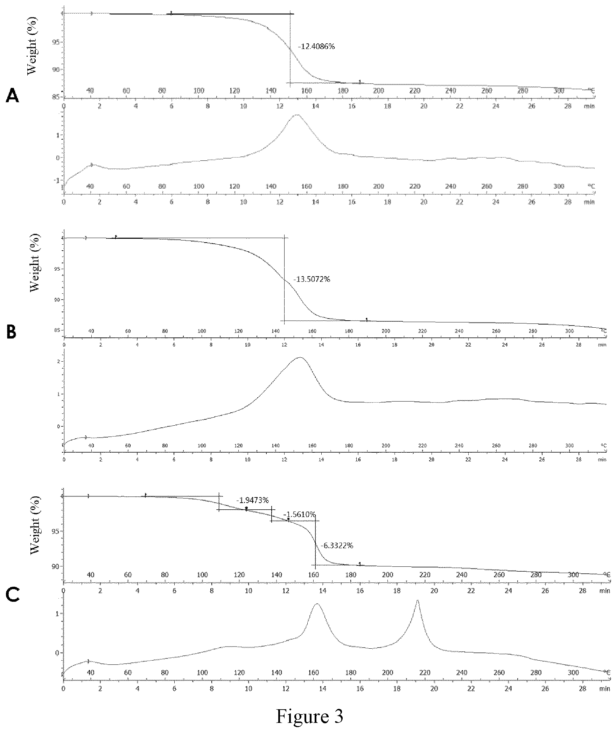

In a specific implementation, the crystalline form 04 of SAGE-217 is also characterized by having a DSC curve as depicted in A of Figure 2 and a TGA curve as depicted in A of Figure 3, wherein the DSC curve shows a broad endothermic peak at 120 °C to 160 °C (starting at 113.9 °C), and above 173.3 °C (starting at 167.5 °C) sample melting occurs. The TGA curve shows a weight loss of 12.4% between 85 - 190 °C, and the EGA spectrum confirms the release of formic acid and ethyl formate, presumably a 1:1 formic acid solvate, and the combined formic acid should come from the degradation of the formate solvent.

In a specific implementation, the crystalline form 04 of SAGE-217 is also characterized by having a DSC curve as depicted in B of Figure 2 and a TGA curve as depicted in B of Figure 3, wherein the DSC curve shows a broad endothermic peak at 100 °C to 180 °C. The TGA curve shows a weight loss of 13.5% between 60 to 190 °C, and the EGA spectrum confirms the release of formic acid and ethyl formate, presumably a 1:1 formic acid solvate, and the combined formic acid should come from the degradation of the formate solvent.

In a specific implementation, the crystalline form 04 of SAGE-217 is characterized by having a DSC curve as depicted in C of Figure 2 and a TGA curve as depicted in C of Figure 3, wherein the DSC curve shows a broad endothermic peak at 110.5 °C (starting at 98.5 °C), a gentler endothermic peak at 147 °C (starting at 144 °C) and a sharp endothermic peak at 155.5 °C (starting at 152.5°C). Above 208.8 °C (starting at 205.7 °C), sample melting occurs. The TGA curve shows that three different and continuous weight loss between 70 - 185 °C are 1.9%, 1.6% and 6.3%, respectively, and the EGA spectrum confirms that formic acid is released. It is detected in EGA that the released formic acid accounts for 9.8% of the total weight of the sample. By calculation, the molar ratio of SAGE-217 to formic acid is 1: 0.97, indicating that a 1:1 formic acid solvate is formed.

The second solution of the present application is: a crystalline form 06 of SAGE-217 has an X-ray powder diffraction pattern with characteristic peaks at 2theta values of 8.7±0.2°, 10.0±0.2°, 13.2±0.2°, 15.0±0.2°, 15.8±0.2°, and 17.3±0.2°.

Further, it has an X-ray powder diffraction pattern with characteristic peaks at 2theta values of 5.0±0.2°, 5.5±0.2°, 19.4±0.2°, 20.0±0.2°, and 21.9±0.2°.

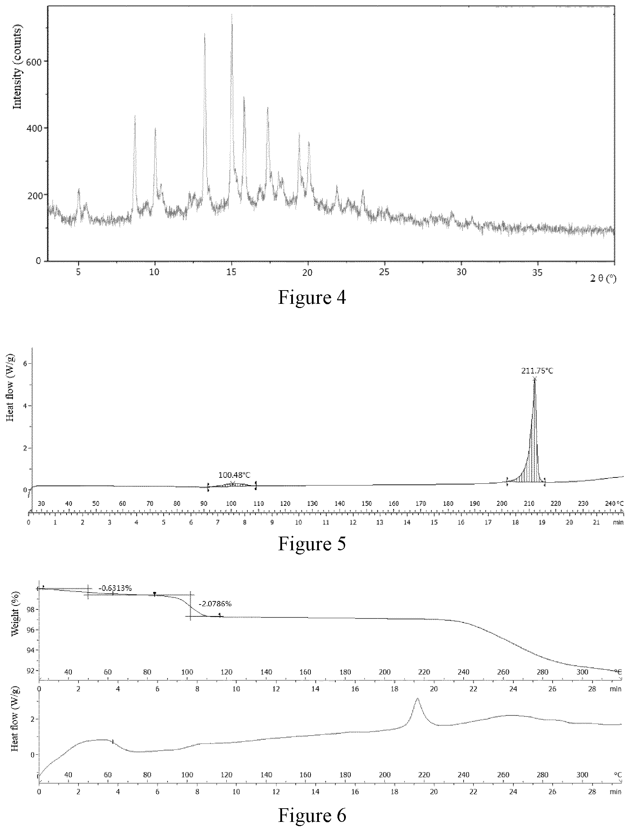

According to some specific implementations, the crystalline form 06 has an X-ray powder diffraction pattern substantially as depicted in Figure 4.

In another aspect, this application provides a method for preparing the crystalline form 06 of SAGE-217 by cooling or evaporative crystallization using nitromethane as a solvent.

In a specific implementation, the crystalline form 06 of crystalline SAGE-217 may be prepared by crystallization by the methods reported in Examples 6 to 7.

The crystalline form 06 remains stable in the exposed powder state for 16 hours, and has a stability of more than seven days when stored in a sealed vial at 25 °C/45% RH.

In a specific implementation, the crystalline form 06 of SAGE-217 is characterized by having a DSC curve as depicted in Figure 5 and a TGA curve as depicted in Figure 6. The DSC curve of crystalline form 06 shows that there are two endothermic peaks at a temperature lower than 240 °C: one is at 93.2 °C formed due to the heat absorption of solvent release, and the other one is at T of 209.4 °C due to the heat absorption of melting. TGA shows that the first weight loss was due to the release of water, which is 0.6% from 25 °C to 95 °C; the second weight loss is 2.1%, which is consistent with the release of nitromethane detected by EGA. By calculation, the molar ratio of SAGE-217 to nitromethane is 1: 0.14, indicating that the captured solvent may form a non-stoichiometric solvate. The compound will degrade above 220 °C.

The third solution of the present application is: a crystalline form D-1 of SAGE-217 has an X-ray powder diffraction pattern with characteristic peaks at 2theta values of 7.2±0.2°, 8.6±0.2°, 13.3±0.2°, 19.6±0.2°, and 23.0±0.2°.

Further, it has an X-ray powder diffraction pattern with characteristic peaks at 2theta values of 7.9±0.2°, 10.6±0.2°, 15.7±0.2°, 16.3±0.2°, 21.3±0.2°, and 21.6±0.2°.

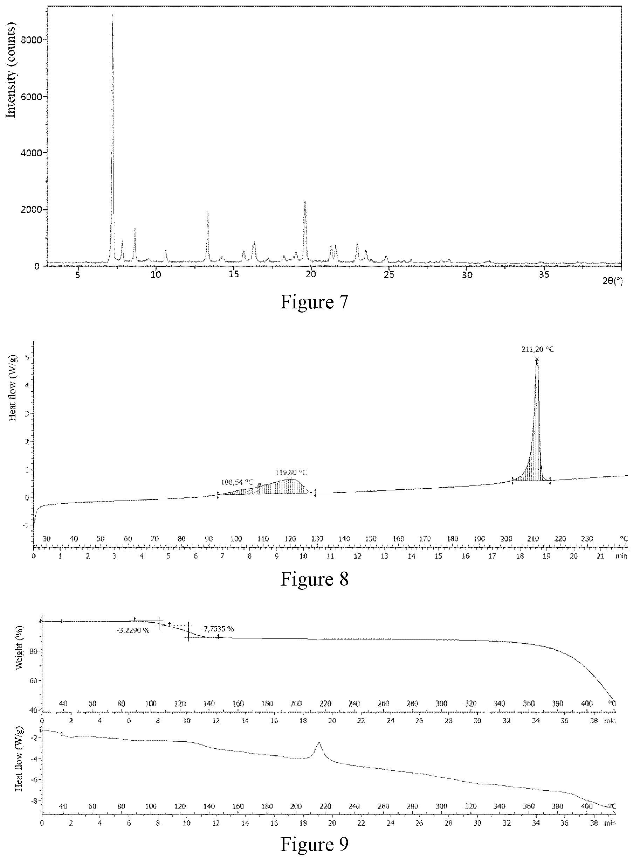

According to some specific implementations, the crystalline form D-1 has an X-ray powder diffraction pattern substantially as depicted in Figure 7.

In another aspect, this application provides a method for preparing the crystalline form D-1 of SAGE-217 by cooling or evaporative crystallization using 4-methyl-2-pentanone as a solvent.

In a specific implementation, the crystalline form D-1 of crystalline SAGE-217 may be prepared by crystallization by the methods reported in Examples 8 to 10.

The crystalline form D-1 may be prepared by evaporative crystallization of 4-methyl-2-pentanone. It is crystallized by evaporation of 4-methyl-2-pentanone solution at room temperature to 40 °C under reduced pressure. The crystalline form D-1 remains stable in the exposed powder state for 18 hours, and has a stability of more than seven days when stored in a sealed vial at 25 °C/45% RH. In 9 months of storage at a temperature of 0 - 8 °C, the chemical stability is good, and the crystalline form remains.

In a specific implementation, the crystalline form D-1of SAGE-217 is characterized by having a DSC curve as depicted in Figure 8 and a TGA curve as depicted in Figure 9. The DSC curve of crystalline form D-1 shows that there are three endothermic peaks, among which the two endothermic peaks at 93 °C and 99.6 °C are due to the heat absorption caused by solvent release. The endothermic peak at 209.2 °C is due to the heat absorption caused by melting. TGA shows two consecutive weight loss, one is 3.2% between 85 °C - 110 °C and the other is 7.8% between 110 °C - 145 °C, which is consistent with the release of 4-methyl-2-pentanone detected by EGA. The total weight loss is 11%, and by calculation, the molar ratio of SAGE-217 to 4-methyl-2-pentanone is 1: 0.5, indicating that a hemisolvate is formed. The compound degrades above 300 °C.

The present disclosure further provides a crystalline form D-2 of SAGE-217, which has an X-ray powder diffraction pattern with characteristic peaks at 2theta values of 7.3±0.2°, 8.6±0.2°, 13.4±0.2°, 19.7±0.2°, and 23.3±0.2°.

More further, it has an X-ray powder diffraction pattern with characteristic peaks at 2theta values of 7.8±0.2°, 10.6±0.2°, 15.5±0.2°, 16.4±0.2°, 19.0±0.2°, and 21.3±0.2°.

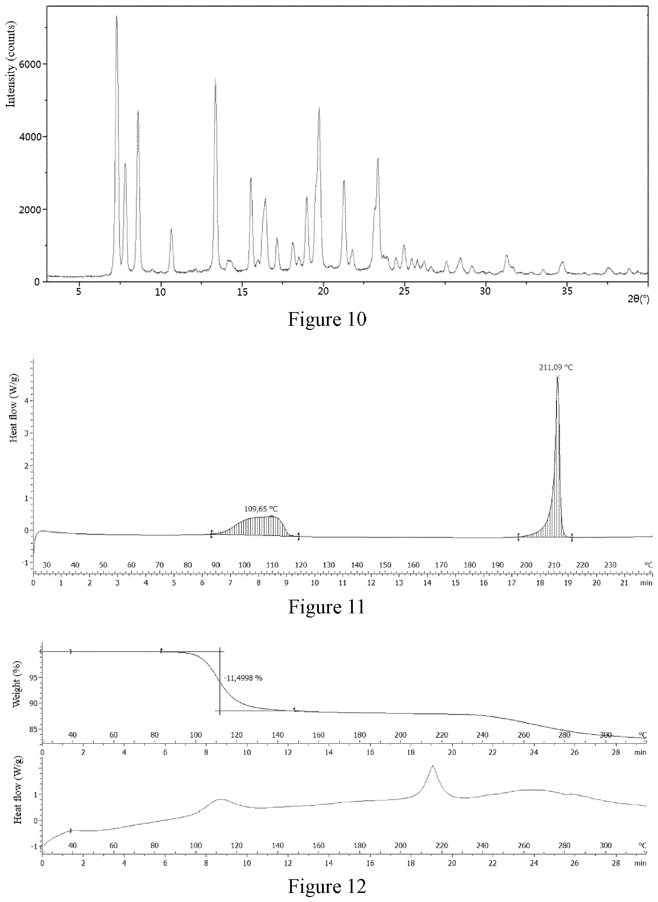

According to some specific implementations, the crystalline form D-2 has an X-ray powder diffraction pattern substantially as depicted in Figure 10.

In another aspect, this application provides a method for preparing the crystalline form D-2 of SAGE-217 by evaporative crystallization using a mixture of isobutyl acetate and ketone as a solvent.

In some implementations, the crystalline form D-2 of crystalline SAGE-217 may be prepared by crystallization by the methods reported in Examples 11 to 12.

The crystalline form D-2 may be prepared by cooling or evaporative crystallization of the mixed solvent of 4-methyl-2-pentanone/ isobutyl acetate. The crystalline form D-2 remains stable in the exposed powder state for 18 hours, and has a crystalline form stability of more than seven days when stored in a sealed vial at 25 °C/45% RH. In 9 months of storage at a temperature of 0 - 8 °C, the chemical stability is good.

In a specific implementation, the crystalline form D-2 of SAGE-217 is characterized by having a DSC curve as depicted in Figure 11 and a TGA curve as depicted in Figure 12. The DSC curve of crystalline form D-2 shows that there are two endothermic peaks, which are at 92.4°C formed due to the heat absorption of solvent release, and 209.4 °C due to the heat absorption of melting, respectively. TGA shows the weight loss between 85 °C - 150 °C is 11.5%, which is consistent with the release of isobutyl acetate detected by EGA. By calculation, the molar ratio of SAGE-217 to isobutyl acetate is 1: 0.5, indicating that a semisolvate is formed. The compound degrades above 220 °C.

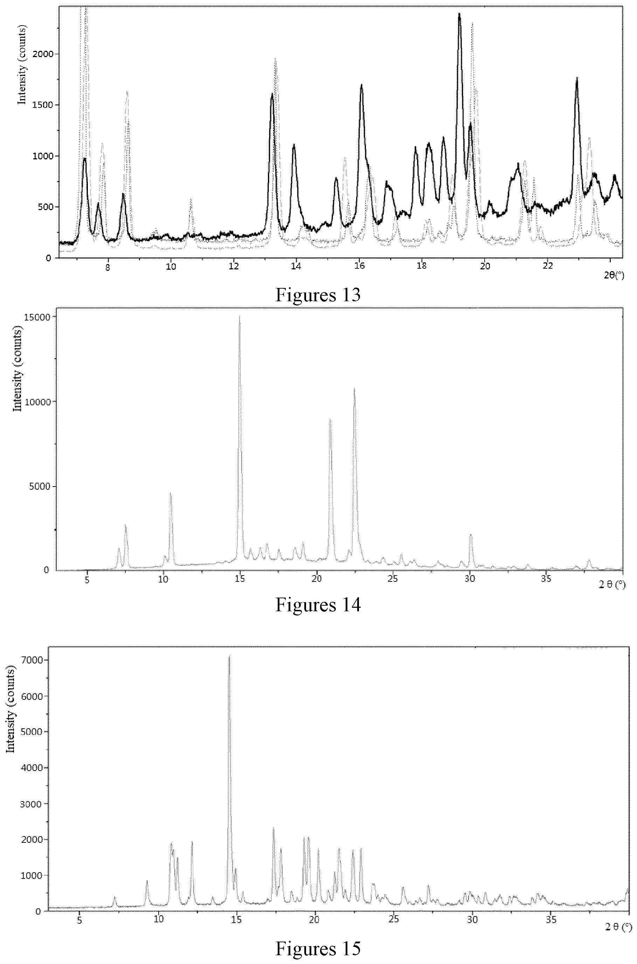

The XRPD patterns of the crystalline form D-1 and crystalline form D-2 of SAGE-217 and the sample of crystalline form D of SAGE-217 separated from the solvent system (tetrahydrofuran/water) in the patent WO2018039378A1 are marked and compared, and the three are considered to be different crystalline forms. As shown in the comparison of the XRPD patterns reported in Figure 13, the crystalline forms D-1 and D-2 described in the present disclosure have significantly different shift signals relative to the crystalline form D in the diffraction pattern, confirming that they are different crystalline forms. In addition, based on EGA data, crystalline form D-1 and crystalline form D-2 are crystalline forms of the semisolvates of 4-methyl-2-pentanone and isobutyl acetate, respectively, and there is no 4-methyl-2-pentanone or isobutyl acetate in the solvent system used for obtaining crystalline form D.

In another specific implementation, crystalline form D-1 and crystalline form D-2 of the SAGE-217 crystal provided by the present disclosure show better stability than crystalline form D separated according to the solvent system reported in WO2018039378A1.

In addition, this application also relates to crystalline form 01, crystalline form 02, crystalline form 03, crystalline form 05, crystalline form 07, crystalline form 08 and crystalline form 09 of SAGE-217. They may be prepared by crystallization by the methods reported in Examples 18 - 24. The crystalline form 01, crystalline form 02, crystalline form 03, crystalline form 05, crystalline form 07, crystalline form 08, and crystalline form 09 of SAGE-217 provided by this application are characterized in the XRPD patterns reported in Figures 14 - 20. The crystalline form has relatively low stability and tends to transform into an anhydrous crystalline form which is more stable.

Compared with the prior art, this application provides more new crystalline forms of SAGE-217, which provides more choices for drug development. Among the new crystalline forms, crystalline form 04, crystalline form 06, crystalline form D-1, crystalline form D-2, etc. also have very good stability, and crystalline form 04 also has significantly improved mechanical stability than existing crystalline forms.

### Brief Description of the Drawings

Figure 1 shows the XRPD patterns of crystalline form 04 of Compound I, where A is the XRPD pattern of the crystalline form 04 obtained in Embodiment 1, B is the XPRD pattern of the crystalline form 04 obtained in Embodiment 2, and C and D are the XRPD patterns of the crystalline form 04 obtained in Embodiments 4 and 5;
Figure 2 shows the DSC curves of crystalline form 04 of Compound I, where A is the DSC curve of the crystalline form 04 obtained in Embodiment 1, B is the DSC curve of the crystalline form 04 obtained in Embodiment 2, and C is the DSC curve of the crystalline form 04 obtained in Embodiment 4;
Figure 3 shows the TGA curves and heat flow curves of crystalline form 04 of Compound I, where A are the TGA curve (upper) and heat flow curve (lower) of the crystalline form 04 obtained in Embodiment 1, B are the TGA curve (upper) and heat flow curve (lower) of the crystalline form 04 obtained in Embodiment 2, and C are the TGA curve (upper) and heat flow curve (lower) of the crystalline form 04 obtained in Embodiment 4;
Figure 4 shows the XRPD pattern of crystalline form 06 of Compound I;
Figure 5 shows the DSC curve of crystalline form 06 of Compound I;
Figure 6 shows the TGA curve (upper) and heat flow curve (lower) of crystalline form 06 of Compound I;
Figure 7 shows the XRPD pattern of crystalline form D-1 of Compound I;
Figure 8 shows the DSC curve of crystalline form D-1 of Compound I;
Figure 9 shows the TGA curve (upper) and heat flow curve (lower) of crystalline form D-1 of Compound I;
Figure 10 shows the XRPD pattern of crystalline form D-2 of Compound I;
Figure 11 shows the DSC curve of crystalline form D-2 of Compound I;
Figure 12 shows the TGA curve (upper) and heat flow curve (lower) of crystalline form D-2 of Compound I;
Figure 13 shows an XRPD comparison diagram of the crystalline forms D-1 and D-2 of compound I and the crystalline form D reported in WO2018039378A1;
Figure 14 shows the XRPD pattern of crystalline form 01 of Compound I;
Figure 15 shows the XRPD pattern of crystalline form 02 of Compound I;
Figure 16 shows the XRPD pattern of crystalline form 03 of Compound I;
Figure 17 shows the XRPD pattern of crystalline form 05 of Compound I;
Figure 18 shows the XRPD pattern of crystalline form 07 of Compound I;
Figure 19 shows the XRPD pattern of crystalline form 08 of Compound I;
Figure 20 shows the XRPD pattern of crystalline form 09 of Compound I;
Figure 21 shows the DVS curve of crystalline form 04 of Compound I;
Figure 22 shows the XRPD patterns of crystalline form 04 before and after applying 2 tons of mechanical pressure;
Figure 23 shows the XRPD patterns of the patented crystalline form K of WO 2018/039378 A1 before and after applying 2 tons of mechanical pressure.

### Detailed Description of Exemplary Embodiments

In the following, the present disclosure is further explained by specific embodiments, but they should not be concluded to limit the protective scope of the present disclosure. Those skilled in the art can make improvements to the preparation method and use apparatus within the scope of the claims, and these improvements should also be considered as within the protection scope of the present disclosure. Therefore, the protection scope of the present patent for an invention should be subject to the appended claims.

In the following embodiments, the test method is usually implemented according to conventional conditions or conditions recommended by the manufacturer; the compound I is prepared by the method of patent WO2014169833.

The explanations of the abbreviations used in the present disclosure are as follows:
XRPD: X-ray powder diffraction
DSC: Differential Scanning Calorimetry
TGA: Thermogravimetric Analysis

The X-ray powder diffraction patterns of the present disclosure were collected on a Rigaku Miniflex 600 X-ray diffraction powder diffractometer.

XRPD scanning parameters: initial position [°2Th.]: 3; end position [°2Th.]: 40; step length of scanning: 0.01°; scanning speed: 10°/min; copper anode (λ=1 .54 Å); voltage: 15 mA; current: 40 kV.

The differential scanning calorimetry (DSC) curves of the present disclosure were collected on a Mettler-Toledo DSC1. The method parameters of differential scanning calorimetry (DSC) were as follows:
Scan range: 25 to 250 °C; Scan rate: 10 °C/min
Purge gas: nitrogen

The thermogravimetric analysis (TGA) curves of the present disclosure were collected on a Mettler-Toledo TGA/DSC1.

The method parameters of thermogravimetric analysis (TGA) were as follows:
Scan range: 25 to 320 °C; Scan rate: 10 °C/min
Purge gas: nitrogen

In the following, the implementation of the present application is described through embodiments, and those skilled in the art should realize that these specific embodiments only show the implemented technical solutions selected to achieve the purpose of this application, and are not limitations on the technical solutions. According to the teachings of this application, it is obvious that the improvement of the technical solution of this application in combination with the existing technology is obvious, and they all fall within the protection scope of the present application.

### Example 1: Preparation of crystalline form 04 of Sage-217

A 10 mg/mL ethyl formate solution of Sage-217 was prepared, the solution was stirred at 25 °C for 60 min to dissolve completely, and filtered with an organic filter head (25mm/0.45µm), and the resulting filtrate stood at 10 °C and was filtered to give a white solid, whose XRPD spectrum is shown as a spectral line A in Figure 1, and the corresponding XRPD data is as follows:

| Position [2θ] (±0.2°) | D spacing [Å] | Relative intensity [%] |
|---|---|---|
| 6.8° | 13.0 | 14.9 |
| 11.6° | 7.6 | 100.0 |
| 13.5° | 6.6 | 47.5 |
| 14.7° | 6.0 | 19.5 |
| 16.2° | 55 | 20.8 |
| 16.5° | 5.4 | 24.3 |
| 18.7° | 4.7 | 20.9 |
| 19.2° | 4.6 | 25.1 |
| 21.3° | 4.2 | 24.5 |
| 23.2° | 3.8 | 33.0 |

### Example 2 (Repeat the operation of Example 1)

A 10 mg/mL ethyl formate solution of Sage-217 was prepared, the solution was stirred at 25 °C for 60 min to dissolve completely, and filtered with an organic filter head (25mm/0.45µm), and the resulting filtrate stood at 10 °C and was filtered to give a white solid, whose XRPD spectrum is shown as a spectral line B in Figure 1.

### Example 3: Preparation of crystalline form 04 of Sage-217

A 25 mg/mL ethyl formate solution of Sage-217 was prepared, the solution was stirred at 25 °C for 6 h to dissolve completely, and filtered with an organic filter head (25mm/0.45µm), and the resulting filtrate stood at 25 °C and was filtered to give a white solid, whose XRPD spectrum is consistent with Figure 1A.

### Example 4: Preparation of crystalline form 04 of Sage-217

A 10mg/mL formic acid/isobutanol (1/1, V/V) solution of Sage-217 was prepared, the solution was stirred at 25 °C for 60 min to dissolve completely, and filtered with an organic filter head (25mm/0.45µm), and the resulting filtrate was crystallized under reduced pressure at 25 °C and was filtered to give a white solid, whose XRPD spectrum is shown as a spectral line C in Figure 1.

### Example 5: Preparation of crystalline form 04 of Sage-217

A 10 mg/mL formic acid/isobutanol (1/1, V/V) solution of Sage-217 was prepared, the solution was stirred at 25 °C for 60 min to dissolve completely, and filtered with an organic filter head (25mm/0.45µm), and the resulting filtrate was crystallized under reduced pressure at 40 °C and was filtered to give a white solid, whose XRPD spectrum is shown as a spectral line D in Figure 1.

### Example 6: Preparation of crystalline form 06 of Sage-217

A 10 mg/mL nitromethane solution of Sage-217 was prepared, the solution was stirred at 25 °C for 60 min to dissolve completely, and filtered with an organic filter head (25mm/0.45µm), and the resulting filtrate was crystallized under reduced pressure at 25 °C and was filtered to give a white solid, whose XRPD spectrum is shown in Figure 4, and the corresponding XRPD data is as follows:

| Position [2θ] (±0.2°) | D spacing [Å] | Relative intensity [%] |
|---|---|---|
| 5.0° | 17.8 | 16.1 |
| 5.5° | 16.0 | 9.3 |
| 8.7° | 10.2 | 52.2 |
| 10.0° | 8.9 | 42.4 |
| 13.2° | 6.7 | 86.9 |
| 15.0° | 5.9 | 100.0 |
| 15.8° | 5.6 | 55.9 |
| 17.3° | 5.1 | 49.1 |
| 19.4° | 4.6 | 34.9 |
| 20.0° | 4.4 | 32.5 |
| 21.9° | 4.1 | 11.4 |

### Example 7: Preparation of crystalline form 06 of Sage-217

A 10 mg/mL nitromethane solution of Sage-217 was prepared, the solution was stirred at 25 °C for 60 min to dissolve completely, and filtered with an organic filter head (25mm/0.45µm), and the resulting filtrate was crystallized under reduced pressure at 40 °C and was filtered to give a white solid, whose XRPD spectrum is shown in Figure 4.

### Example 8: Preparation of crystalline form D-1 of Sage-217

A 10 mg/mL 4-methyl-2-pentanone solution of Sage-217 was prepared, the solution was stirred at 25 °C for 60 min to dissolve completely, and filtered with an organic filter head (25mm/0.45µm), and the resulting filtrate was crystallized under reduced pressure at 25 °C and was filtered to give a white solid, whose XRPD spectrum is shown in Figure 7, and the corresponding XRPD data is as follows:

| Position [2θ] (±0.2°) | D spacing [Å] | Relative intensity [%] |
|---|---|---|
| 7.2° | 12.3 | 100.0 |
| 7.9° | 11.3 | 8.1 |
| 8.6° | 10.2 | 13.0 |
| 10.6° | 8.3 | 4.7 |
| 13.3° | 6.6 | 19.8 |
| 15.7° | 5.7 | 3.7 |
| 16.3° | 5.4 | 7.6 |
| 19.6° | 4.5 | 23.5 |
| 21.3° | 4.2 | 6.3 |
| 21.6° | 4.1 | 7.1 |
| 23.0° | 3.9 | 7.0 |

### Example 9: Preparation of crystalline form D-1 of Sage-217

A 10 mg/mL 4-methyl-2-pentanone solution of Sage-217 was prepared, the solution was stirred at 25 °C for 60 min to dissolve completely, and filtered with an organic filter head (25mm/0.45µm), and the resulting filtrate was crystallized under reduced pressure at 40 °C and was filtered to give a white solid, whose XRPD spectrum is shown in Figure 7.

### Example 10 (Repeat the operation of Example 9)

A 10 mg/mL 4-methyl-2-pentanone solution of Sage-217 was prepared, the solution was stirred at 25 °C for 60 min to dissolve completely, and filtered with an organic filter head (25mm/0.45µm), and the resulting filtrate was crystallized under reduced pressure at 40 °C and was filtered to give a white solid, whose XRPD spectrum is shown in Figure 7.

### Example 11: Preparation of crystalline form D-2 of Sage-217

A 10 mg/mL 4-methyl-2-pentanone/isobutyl acetate (1/1, v/v) solution of Sage-217 was prepared, the solution was stirred at 25 °C for 60 min to dissolve completely, and filtered with an organic filter head (25mm/0.45µm), and the resulting filtrate was crystallized under reduced pressure at 25 °C and was filtered to give a white solid, whose XRPD spectrum is shown in Figure 10, and the corresponding XRPD data is as follows:

| Position [2θ] (±0.2°) | D spacing [Å] | Relative intensity [%] |
|---|---|---|
| 7.3° | 12.2 | 100.0 |
| 7.8° | 11.3 | 42.1 |
| 8.6° | 10.3 | 63.5 |
| 10.6° | 8.3 | 18.2 |
| 13.4° | 6.6 | 79.3 |
| 15.5° | 5.7 | 32.6 |
| 16.4° | 5.4 | 28.9 |
| 19.0° | 4.7 | 28.1 |
| 19.7° | 4.5 | 66.3 |
| 21.3° | 4.2 | 35.1 |
| 23.3° | 3.8 | 44.6 |
| 31.3° | 2.9 | 7.0 |

### Example 12: Preparation of crystalline form D-2 of Sage-217

A 10 mg/mL 4-methyl-2-pentanone/isobutyl acetate (1/1, v/v) solution of Sage-217 was prepared, the solution was stirred at 25 °C for 60 min to dissolve completely, and filtered with an organic filter head (25mm/0.45µm), and the resulting filtrate was crystallized under reduced pressure at 40 °C and was filtered to give a white solid, whose XRPD spectrum is shown in Figure 10.

### Example 13: DVS test of crystalline form 04 of Sage-217

The sample of crystalline form 04 prepared in Example 1 was placed on a microbalance plate in a sealed sample chamber, and then exposed to different relative humidity, the relative humidity ranged from 0% or 40% to 90%, and changed in 10% increments. At each humidity level, the sample is balanced when dm/dt is less than 0.002% within 10 minutes. The mass of the dried sample and the equilibrium mass at each humidity level were recorded, and the weight change was plotted against relative humidity, which is the moisture adsorption isotherm of the sample. See Figure 15 for the results.

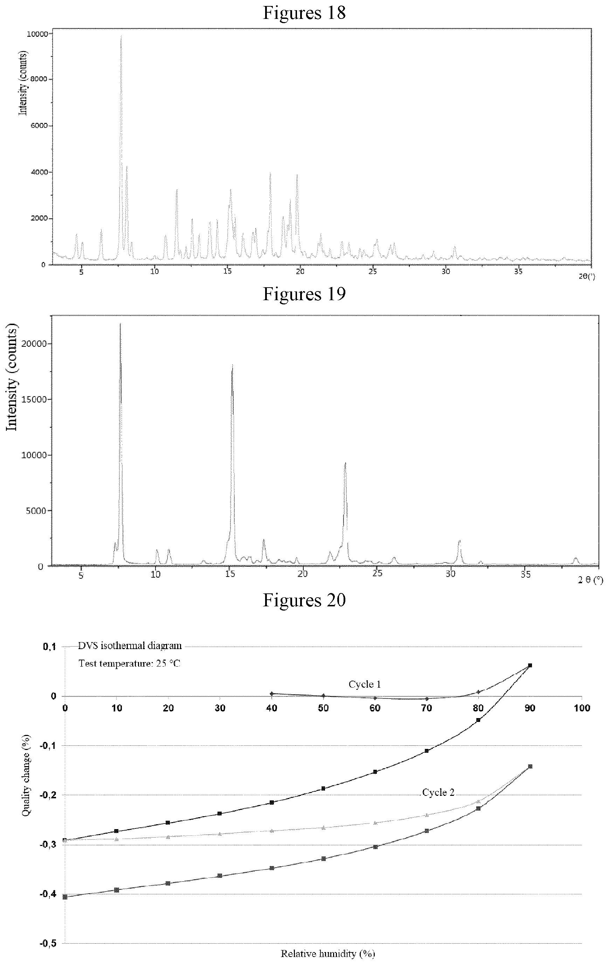

It can be seen from Figure 21 that when the relative humidity changed from 40% to 90% in the crystalline form 04 at 25 °C, the water absorption of the crystalline form 04 is 0.06%, indicating that the crystalline form 04 has no or almost no hygroscopicity.

Regarding the description of hygroscopicity characteristics and the definition of hygroscopic weight gain (Appendix XIX J of Chinese Pharmacopoeia 2010 Edition, Guidelines for Hygroscopicity, with experimental conditions: 25±1 °C, 80% relative humidity):
Deliquescent: sufficient water is absorbed to form liquid
Extremely hygroscopic: the increase in mass is not less than 15%
Hygroscopic: the increase in mass is less than 15% but not less than 2%
Slightly hygroscopic: the increase in mass is less than 2% but not less than 0.2%
Practical nonhygroscopic: the increase in mass is less than 0.2%.

### Example 14: Stability of crystalline form 04 under different temperature and humidity conditions

The samples of crystalline form 04 prepared in Example 1 were taken, and placed at 25 °C/60% humidity and 40 °C/75% humidity for 1 month; placed at 2 - 8°C for 9 months; after the placement, the above-mentioned samples were taken out and tested for the crystalline form. The results are shown in the following table.

| | | | |
|---|---|---|---|
| Temperature | 25 °C | 40 °C | 2-8 °C (in freezer) |
| Relative humidity | 60% | 75% | / |
| Storage time | 1 month | 1 month | 9 months |
| Crystalline form | Crystalline form 04 | Crystalline form 04 | Crystalline form 04 |

Samples of the crystalline form 04 of the present disclosure were taken and placed at 2 - 8 °C for 9 months, and performed purity determination by high performance liquid chromatography before and after placement to evaluate chemical stability. The purity results of high performance liquid chromatography are shown in the following table. The results show that the crystalline form 04 has good chemical stability.

| Test time | HPLC purity | |
|---|---|---|
| | Example 1 | Example 2 |
| Before storage | 97.7% | 98.5% |
| After storage for 9 months | 97.5% | 98.2% |

### Example 15: Stability experiment of crystalline form D-1

The crystalline form D-1 prepared in Example 8 was placed at 2 - 8 °C, and after 9 months of storage, it was taken out to evaluate chemical stability, and the results showed that the crystalline form did not change. Samples of the crystalline form D-1 prepared by the present disclosure were taken and placed at 2 - 8 °C for 9 months, and performed purity determination by high performance liquid chromatography before and after placement to evaluate chemical stability. The purity results of high performance liquid chromatography are shown in the following table. The results show that the crystalline form 06 has good chemical stability.

| Test time | HPLC purity | |
|---|---|---|
| | Example 8 | Example 9 |
| Before storage | 99.1% | 99.4% |
| After storage for 9 months | 98.8% | 99.2% |

### Example 16: Stability experiment of crystalline form D-2

Samples of the crystalline form D-2 prepared by the present disclosure were taken and placed at 2 - 8 °C for 9 months, and performed purity determination by high performance liquid chromatography before and after placement to evaluate chemical stability. The purity results of high performance liquid chromatography are shown in the following table. In 9 months of storage at a temperature of 0 - 8 °C, the chemical stability is good.

| Test time | HPLC purity | |
|---|---|---|
| | Example 11 | Example 12 |
| Before storage | 99.1% | 99.2% |
| After storage for 9 months | 98.7% | 98.9% |

### Example 17: Thermodynamic solubility and mechanical pressure stability tests of crystalline form 04

Thermodynamic solubility test: the solid powder was added to water to a saturated solution, and then the suspension liquid was stirred under magnetic stirring at 25 °C for 24 hours. After completion, it was filtered with a 0.20 µm filter and analyzed by HPLC. Tests were carried out in parallel, and each group was tested twice to take the average.

| Group | Crystalline form 04 | | Crystalline form K | |
|---|---|---|---|---|
| | Area (mAu^{∗}s) | Concentration (ng/mL) | Area (mAu^{∗}s) | Concentration (ng/mL) |
| Group 1 | 12.6 | 450.6 | 10.9 | 392.7 |
| Group 2 | 11.8 | 424.3 | 11.2 | 403.2 |
| Average | 12.2 | 437.5 | 11.1 | 398.0 |

The thermodynamic dissolution results are recorded in the table above. Based on the results of these samples, the thermodynamic solubility was calculated. The value of crystalline form 04 is 437.5 ng/mL, the value of crystalline form K is 398.0 ng/mL, and the thermodynamic solubility of the two was within the error range, and the results were basically the same.

To further evaluate the mechanical stability of the crystalline form 04 of the present application and the crystalline form K reported in WO 2018/039378 A1, a 100 mg sample was subjected to 2 tons of mechanical pressure, and the recovered tablets were gently ground and tested by XRPD.

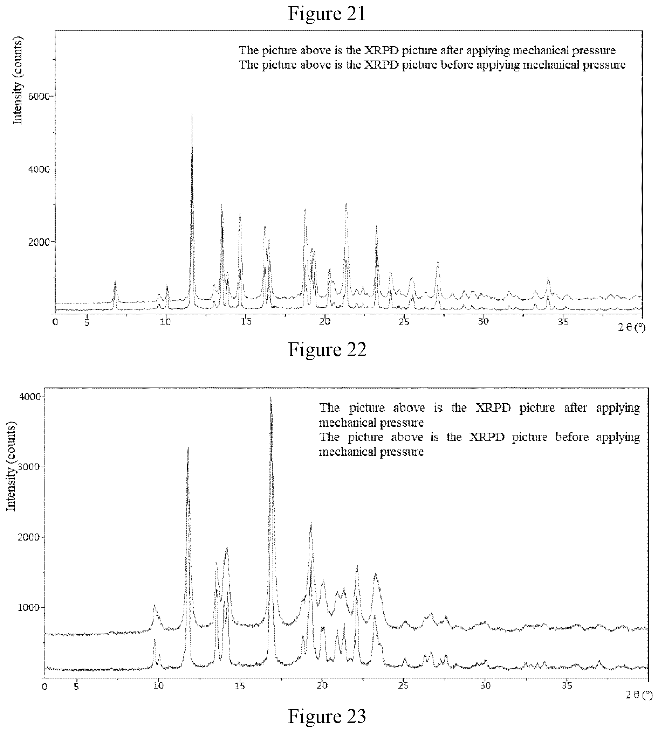

The XRPD test of crystalline form 04 showed no obvious change (Figure 22), while for the reported crystalline form K in WO 2018/039378 A1, a slight decrease in its crystallinity was observed (Figure 23). From one side, it shows that the physical stability of the crystalline form 04 of this application is better than that of the crystalline form K.

### Example 18: Preparation of crystalline form 01 of Sage-217

A 10 mg/mL p-xylene solution of Sage-217 was prepared, cooled from 75 °C to 25 °C, stirred to crystallize, filtered to give a solid, which was tested by XRPD, the XRPD pattern is shown in Figure 14, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.5 | 11.8 | 17.4 |
| 10.4 | 8.5 | 29.9 |
| 10.6 | 8.4 | 13.5 |
| 14.9 | 5.9 | 100.0 |
| 15.1 | 5.9 | 40.1 |
| 20.9 | 4.3 | 58.8 |
| 22.4 | 4.0 | 67.2 |
| 22.5 | 4.0 | 69.8 |
| 30.0 | 3.0 | 13.9 |
| 30.1 | 3.0 | 13.1 |

### Example 19: Preparation of crystalline form 02 of Sage-217

A 10 mg/mL ethanediamine solution of Sage-217 was prepared, cooled from 50 °C to 25 °C under stirring to crystallize, and filtered to give a solid and dried to test by XRPD, the XRPD pattern is shown in Figure 15, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.2 | 12.2 | 4.3 |
| 9.3 | 9.5 | 10.3 |
| 10.8 | 8.2 | 26.0 |
| 11.0 | 8.1 | 24.2 |
| 11.2 | 7.9 | 19.6 |
| 12.1 | 7.3 | 27.0 |
| 14.5 | 6.1 | 100.0 |
| 17.3 | 5.1 | 33.0 |
| 17.7 | 5.0 | 21.9 |
| 19.3 | 4.6 | 25.8 |
| 19.5 | 4.5 | 27.4 |
| 20.2 | 4.4 | 22.9 |
| 21.5 | 4.1 | 20.8 |
| 22.4 | 4.0 | 22.4 |
| 22.9 | 3.9 | 22.0 |

### Example 20: Preparation of crystalline form 03 of Sage-217

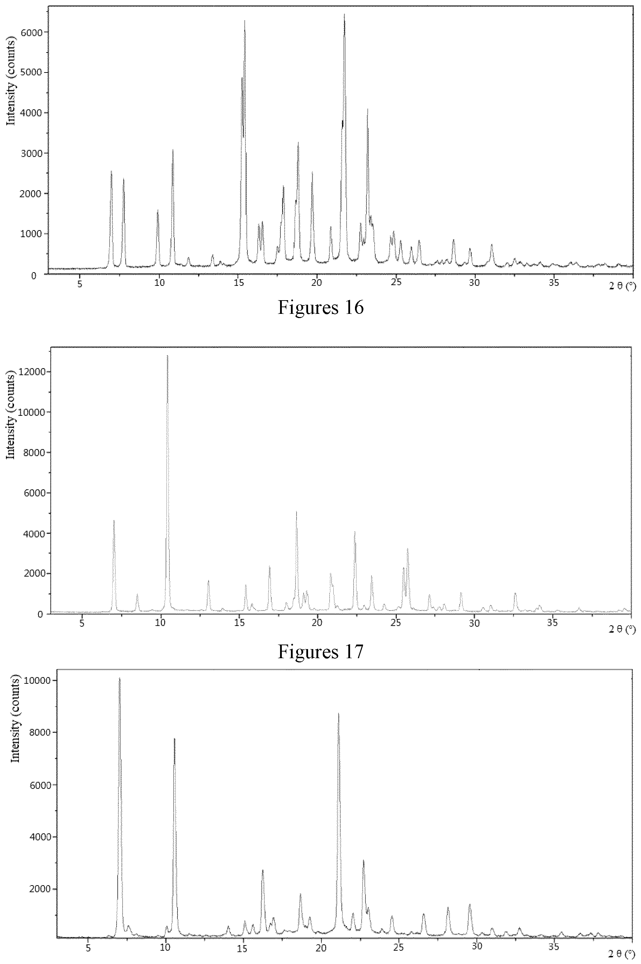

A 10 mg/mL diethyl carbonate solution of Sage-217 was prepared, cooled from 75 °C to 25 °C, stirred to crystallize, filtered to give a solid, which was tested by XRPD, the XRPD pattern is shown in Figure 16, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.0 | 12.6 | 38.1 |
| 7.7 | 11.4 | 36.3 |
| 9.9 | 8.9 | 23.4 |
| 10.9 | 8.2 | 46.3 |
| 15.3 | 5.8 | 76.5 |
| 15.4 | 5.7 | 100.0 |
| 16.6 | 5.4 | 16.3 |
| 17.9 | 5.0 | 31.0 |
| 18.8 | 4.7 | 49.0 |
| 21.7 | 4.1 | 97.7 |
| 23.2 | 3.8 | 62.7 |

### Example 21: Preparation of crystalline form 05 of Sage-217

A 10 mg/mL glycol dimethyl ether slurry of Sage-217 was prepared, stirred at 25 °C for 15 days, filtered to give a solid, which was tested by XRPD, the XRPD pattern is shown in Figure 17, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.0 | 12.6 | 36.6 |
| 8.5 | 10.4 | 6.5 |
| 10.4 | 8.5 | 100.0 |
| 13.0 | 6.8 | 12.2 |
| 15.4 | 5.8 | 9.9 |
| 16.9 | 5.2 | 17.7 |
| 18.6 | 4.8 | 38.4 |
| 20.8 | 4.3 | 14.1 |
| 22.3 | 4.0 | 30.7 |
| 23.4 | 3.8 | 13.6 |
| 25.7 | 3.5 | 24.4 |
| 25.8 | 3.5 | 20.0 |

### Example 22: Preparation of crystalline form 07 of Sage-217

A 10 mg/mL methylbenzene solution of Sage-217 was prepared, cooled from 75 °C to 10 °C, stirred to crystallize, filtered to give a solid, which was tested by XRPD, the XRPD pattern is shown in Figure 18, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.0 | 12.6 | 100.0 |
| 10.5 | 8.4 | 77.5 |
| 16.2 | 5.5 | 25.6 |
| 18.6 | 4.8 | 14.8 |
| 21.1 | 4.2 | 83.4 |
| 22.7 | 3.9 | 21.7 |
| 26.5 | 3.4 | 8.5 |
| 26.6 | 3.4 | 6.8 |
| 28.2 | 3.2 | 11.2 |
| 29.6 | 3.0 | 12.5 |

### Example 23: Preparation of crystalline form 08 of Sage-217

A 10 mg/mL methylbenzene solution of Sage-217 was prepared, rapidly cooled from 75°C to 10 °C, stirred to crystallize, filtered to give a solid, which was tested by XRPD, the XRPD pattern is shown in Figure 19. The corresponding XRPD data is as follows.

| Position [2θ] (±0.2°) | D spacing [Å] | Relative intensity [%] |
|---|---|---|
| 4.6 | 19.0 | 10.7 |
| 5.0 | 17.6 | 7.6 |
| 6.3 | 13.9 | 13.6 |
| 7.7 | 11.5 | 100.0 |
| 8.1 | 10.9 | 41.4 |
| 10.7 | 8.3 | 10.2 |
| 11.5 | 7.7 | 30.0 |
| 12.6 | 7.0 | 17.1 |
| 13.1 | 6.8 | 10.4 |
| 13.7 | 6.5 | 15.2 |
| 13.8 | 6.4 | 15.0 |
| 14.3 | 6.2 | 17.6 |
| 15.1 | 5.9 | 23.1 |
| 15.2 | 5.8 | 30.5 |
| 15.5 | 5.7 | 19.0 |
| 16.0 | 5.5 | 10.1 |
| 16.7 | 5.3 | 10.5 |
| 17.0 | 5.2 | 12.9 |
| 17.8 | 5.0 | 11.6 |
| 17.9 | 4.9 | 37.3 |
| 18.8 | 4.7 | 17.7 |
| 18.9 | 4.7 | 15.1 |
| 19.1 | 4.6 | 14.3 |
| 19.3 | 4.6 | 25.9 |
| 19.7 | 4.5 | 36.7 |
| 21.4 | 4.1 | 10.5 |
| 25.3 | 3.5 | 7.9 |

### Example 24: Preparation of crystalline form 09 of Sage-217

A 10 mg/mL acetonitrile solution of Sage-217 was prepared, cooled from 50 °C to 25 °C, stirred to crystallize, filtered to give a solid, which was tested by XRPD, the XRPD pattern is shown in Figure 20, and the corresponding XRPD data is as follows. In practice, it is found that the stability of this crystalline form is poorer than the aforementioned crystalline form 04, crystalline form 06, crystalline form D-1, and crystalline form D-2.

| Position [2θ] (±0.2°) | D spacing [Å] | Relative intensity [%] |
|---|---|---|
| 7.6 | 11.6 | 100.0 |
| 7.8 | 11.4 | 43.0 |
| 15.2 | 5.8 | 78.5 |
| 15.3 | 5.8 | 36.9 |
| 17.3 | 5.1 | 10.1 |
| 22.8 | 3.9 | 37.7 |
| 22.9 | 3.9 | 29.2 |
| 30.6 | 2.9 | 10.2 |

This application includes but is not limited to the above embodiments. Any equivalent substitution or partial improvement made under the principle of the spirit of this application will be considered to be within the protection scope of this application.

## Claims

1. A crystalline form 04 of a compound SAGE-217, the chemical name of SAGE-217 being 1-(2-((3R,5R,8R,9R,10S,13S,14S,17S)-3-hydroxy-3,13-dimethylhexadecahydro-lH-cyclopenta[a]phenanthren-17-yl)-2-oxoe thyl)-lH-pyrazole-4- carbonitrile, is **characterized in that**, it has an X-ray powder diffraction pattern with characteristic peaks at 2theta values of 11.6±0.2°, 13.5±0.2°, 16.2±0.2°, 16.5±0.2°, and 23.2±0.2°.

2. The crystalline form 04 according to claim 1, is **characterized in that**, it has an X-ray powder diffraction pattern with characteristic peaks at 2theta values of 6.8±0.2°, 14.7±0.2°, 18.7±0.2°, 19.2±0.2°, and 21.3±0.2°.

3. The crystalline form 04 according to claim 1, is **characterized in that**, it has an X-ray powder diffraction pattern substantially as depicted in Figure 1.

4. The crystalline form 04 according to claim 1, is **characterized in that**, the crystalline form 04 is a formic acid solvate of compound SAGE-217.

5. A preparation method for the crystalline form 04 of compound SAGE-217 according to any one of claims 1 to 4, is **characterized in that**, it comprises crystallizing SAGE-217 in a formate or a mixed system of formic acid and an organic solvent to obtain the crystalline form 04.

6. The preparation method according to claim 5, is **characterized in that**, the ester group of the formate is selected from groups containing C1-C10, and the organic solvent is selected from alcohols containing C1-C10, dichloromethane and acetonitrile.

7. A crystalline form 06 of a compound SAGE-217, the chemical name of SAGE-217 being 1-(2-((3R,5R,8R,9R,10S,13S,14S,17S)-3-hydroxy-3,13-dimethylhexadecahydro-lH-cyclopenta[a]phenanthren-17-yl)-2-oxoe thyl)-lH-pyrazole-4- carbonitrile, is **characterized in that**, it has an X-ray powder diffraction pattern with characteristic peaks at 2theta values of 8.7±0.2°, 10.0±0.2°, 13.2±0.2°, 15.0±0.2°, 15.8±0.2°, and 17.3±0.2°.

8. The crystalline form 06 according to claim 7, is **characterized in that**, it has an X-ray powder diffraction pattern with characteristic peaks at 2theta values of 5.0±0.2°, 5.5±0.2°, 19.4±0.2°, 20.0±0.2°, and 21.9±0.2°.

9. The crystalline form 06 according to claim 7, is **characterized in that**, it has an X-ray powder diffraction pattern substantially as depicted in Figure 4.

10. A preparation method for the crystalline form 06 of compound SAGE-217 according to any one of claims 7 to 9, is **characterized in that**, it comprises crystallizing SAGE-217 in a nitromethane system to obtain the crystalline form 06.

11. A crystalline form D-1 of a compound SAGE-217, the chemical name of SAGE-217 being 1-(2-((3R,5R,8R,9R,10S,13S,14S,17S)-3-hydroxy-3,13-dimethylhexadecahydro-lH-cyclopenta[a]phenanthren-17-yl)-2-oxoe thyl)-lH-pyrazole-4- carbonitrile, is **characterized in that**, it has an X-ray powder diffraction pattern with characteristic peaks at 2theta values of 7.2±0.2°, 8.6±0.2°, 13.3±0.2°, 19.6±0.2°, and 23.0±0.2°.

12. The crystalline form D-1 according to claim 11, is **characterized in that**, it has an X-ray powder diffraction pattern with characteristic peaks at 2theta values of 7.9±0.2°, 10.6±0.2°, 15.7±0.2°, 16.3±0.2°, 21.3±0.2°, and 21.6±0.2°.

13. The crystalline form D-1 according to claim 11, is **characterized in that**, it has an X-ray powder diffraction pattern substantially as depicted in Figure 7.

14. The crystalline form D-1 according to claim 11, is **characterized in that**, the crystalline form D-1 is a 4-methyl-2-pentanone solvate of compound SAGE-217.

15. A preparation method for the crystalline form D-1 of compound SAGE-217 according to any one of claims 11 to 14, is **characterized in that**, it comprises crystallizing SAGE-217 in a 4-methyl-2-pentanone system to obtain the crystalline form D-1.

16. A crystalline form D-2 of a compound SAGE-217, the chemical name of SAGE-217 being 1-(2-((3R,5R,8R,9R,10S,13S,14S,17S)-3-hydroxy-3,13-dimethylhexadecahydro-lH-cyclopenta[a]phenanthren-17-yl)-2-oxoe thyl)-lH-pyrazole-4- carbonitrile, is **characterized in that**, it has an X-ray powder diffraction pattern with characteristic peaks at 2theta values of 7.3±0.2°, 8.6±0.2°, 13.4±0.2°, 19.7±0.2°, and 23.3±0.2°.

17. The crystalline form D-2 according to claim 16, is **characterized in that**, it has an X-ray powder diffraction pattern with characteristic peaks at 2theta values of 7.8±0.2°, 10.6±0.2°, 15.5±0.2°, 16.4±0.2°, 19.0±0.2°, and 21.3±0.2°.

18. The crystalline form D-2 according to claim 16, is **characterized in that**, it has an X-ray powder diffraction pattern substantially as depicted in Figure 10.

19. A preparation method for the crystalline form D-2 of compound SAGE-217 according to any one of claims 16 to 18, is **characterized in that**, it comprises crystallizing SAGE-217 in a mixed system of isobutyl acetate and an organic solvent to obtain the crystalline form D-2.

20. The preparation method according to claim 19, is **characterized in that**, the organic solvent is selected from ketones containing C3-C6.

21. A pharmaceutical composition, containing an active ingredient, is **characterized in that**: the active ingredient comprises a crystalline form of a compound SAGE-217 according to any one of claims 1 - 4, 7 - 9, 11 - 14, and 16 - 18.
